# EUROPÄISCHE PATENTSCHRIFT

(11) **EP 0 508 955 B1**
(45) Veröffentlichungstag und Bekanntmachung des Hinweises auf die Patenterteilung: **19.06.1996**
(21) Anmeldenummer: 92810257.3
(22) Anmeldetag: 03.04.1992
(51) Int. Cl.: A61K 31/425

(54) **Verwendung von 2-Iminothiazolidin-4-onderivaten als neuartige Arzneimittelwirkstoffe**
Use of 2-iminothiazolidin-4-one derivatives as new medicaments
Utilisation du dérivée de 2-iminothiazolidin-4-on comme médicament nouveau

(30) Priorität: 12.04.1991 CH 1087/91
(43) Veröffentlichungstag der Anmeldung: 14.10.1992
(73) Patentinhaber: CIBA-GEIGY AG, CH-4002 Basel (CH)
(72) Erfinder: Feige, Ulrich, Dr., CH-4125 Riehen (CH); Wiesenberg, Irmgard, Dr., W-7858 Weil am Rhein (DE); Widler, Leo, Dr., CH-4142 Münchenstein (CH); Ferrini, Pier Giorgio, Dr., CH-4102 Binningen (CH); Missbach, Martin, Dr., CH-4310 Rheinfelden (CH)

(56) Entgegenhaltungen:
- CH-A- 511 877
- CH-A- 594 648
- DE-A- 2 632 745

## Beschreibung

Die Erfindung betrifft die Verwendung von 2-Iminothiazolidin-4-onderivaten der Formel I 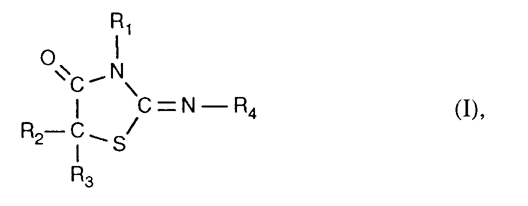
worin R₁ Niederalk-2-en-1-yl, Niederalk-3-en-2-yl, Niederalk-2-in-1-yl oder in 2,3-Stellung durch unter Bildung einer Doppelbindung eliminierbare Reste substituiertes Niederalkyl bedeutet, R₂ Wasserstoff und R₃ unsubstituiertes oder durch zusammen mit Wasserstoff R₂ unter Bildung einer Doppelbindung eliminierbare Reste substituiertes Methyl bedeutet oder R₂ und R₃ beide Wasserstoff oder Niederalkyl bedeuten oder gemeinsam Methylen darstellen und R₄ eine Gruppe der Formeln Ia, Ib oder Ic 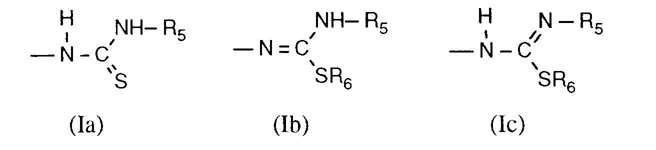
darstellt, in der R₅ Wasserstoff Niederalkyl, Niederalk-2-en-1-yl oder Niederalk-2-in-1-yl und R₆ eine Gruppe der Formel Id oder Ie 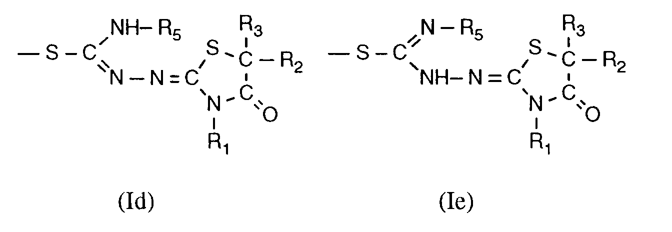
bedeutet, in denen R₁, R₂, R₃ und R₅ die angegebenen Bedeutungen haben, und ihrer pharmazeutisch verwendbaren Salz zur Herstellung pharmazeutischer Präparate für die Behandlung von Erkrankungen des rheumatoiden Formenkreises.

In 2,3-Stellung durch unter Bildung einer Doppelbindung eliminierbare Reste substituiertes Niederalkyl ist beispielsweise 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl.

Durch zusammen mit Wasserstoff R₂ unter Bildung einer Doppelbindung eliminierbare Reste substituiertes Methyl ist beispielsweise Diniederalkylaminomethyl oder Halomethyl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise C₁-C₇-Alkyl, vorzugsweise C₁-C₄-Alkyl, wie insbesondere Methyl oder in zweiter Linie Äthyl, Propyl, Isopropyl oder Butyl, kann aber auch Isobutyl, Sekundärbutyl, Tertiärbutyl oder eine C₅-C₇-Alkyl-, wie Pentyl-, Hexyl- oder Heptylgruppe sein.

Niederalkoxy ist beispielsweise C₁-C₇-Alkoxy, vorzugsweise C₁-C₄-Alkoxy, wie Methoxy, Äthoxy, Propyloxy, Isopropyloxy oder Butyloxy , kann aber auch Isobutyloxy, Sekundärbutyloxy, Tertiärbutyloxy oder eine Pentyloxy-, Hexyloxy- oder Heptyloxygruppe sein.

Niederalk-2-en-1-yl ist beispielsweise C₃-C₇-Alk-2-en-1-yl, insbesondere C₃-C₅-Alk-2-en-1-yl, wie Allyl (Prop-2-en-1-yl) oder Methallyl (2-Methylprop-2-en-1-yl).

Niederalk-3-en-2-yl ist beispielsweise C₃-C₇-Alk-3-en-2-yl, insbesondere C₃-C₅-Alk-3-en-2-yl, wie But-3-en-2-yl.

2- oder 3-Aminoniederalkyl ist beispielsweise 2-Amino-C₃-C₇-alkyl, insbesondere Amino-C₃-C₅-alkyl, wie 2-Aminopropyl oder 2-Amino-2-methyl-propyl, oder 3-Amino-C₃-C₇-alkyl, insbesondere 3-Amino-C₃-C₅-alkyl, wie 3-Aminopropyl oder 3-Amino-2-methyl-propyl.

2- oder 3-Niederalkylaminoniederalkyl ist beispielsweise 2-C₁-C₄-Alkylamino-C₃-C₇-alkyl, insbesondere C₁-C₄-Alkylamino-C₃-C₅-alkyl, wie 2-C₁-C₄-Alkylaminopropyl oder 2-C₁-C₄-Alkylamino-2-methyl-propyl oder 3-C₁-C₄-Alkylamino-C₃-C₇-alkyl, insbesondere 3-C₁-C₄-Alkylamino-C₃-C₅-alkyl, wie 3-C₁-C₄-Alkylaminopropyl oder 3-C₁-C₄-Alkylamino-2-methyl-propyl, wobei C₁-C₄-Alkyl beispielsweise Methyl, Äthyl, Propyl oder Butyl bedeutet.

Diniederalkylaminomethyl ist beispielsweise N,N-Di-C₁-C₄-alkylaminomethyl, wie N,N-Dimethylaminomethyl, N,N-Diäthylaminomethyl, N-Äthyl-N-methyl-aminomethyl, N,N-Dipropylaminomethyl, N-Methyl-N-propyl-aminomethyl, N-Isopropyl-N-methylaminomethyl oder N-Butyl-N-methyl-aminomethyl, kann aber auch N-Isobutyl-N-methyl-aminomethyl, N-Methyl-N-sekundärbutyl-aminomethyl, N-Methyl-N-tertiärbutyl-aminomethyl oder eine N-Methyl-N-pentyl-aminomethyl-, N-Hexyl-N-methylaminomethyl- oder N-Heptyl-N-methyl-aminomethylgruppe sein.

2- oder 3-Diniederalkylaminoniederalkyl ist beispielsweise 2-(N,N-Di-C₁-C₄-alkylamino)-C₃-C₇-alkyl, insbesondere 2-(N,N-Di-C₁-C₄-alkylamino)-C₃-C₅-alkyl, wie 2-(N,N-Di-C₁-C₄-alkylamino)propyl oder 2-(N,N-Di-C₁-C₄-alkylamino)-2-methyl-propyl oder 3-(N,N-Di-C₁-C₄-alkylamino)-C₃-C₇-alkyl, insbesondere 3-(N,N-Di-C₁-C₄-alkylamino)-C₃-C₅-alkyl, wie 3-(N,N-Di-C₁-C₄-alkylamino)propyl oder 3-(N,N-Di-C₁-C₄-alkylamino)-2-methyl-propyl, wobei C₁-C₄-Alkyl beispielsweise Methyl, Äthyl, Propyl oder Butyl bedeutet.

2- oder 3-Niederalkylenaminoniederalkyl ist beispielsweise 4- bis 7-gliedriges 2-(N,N-Alkylenamino)-C₃-C₇-alkyl, insbesondere 2-(N,N-Alkylenamino)-C₃-C₅-alkyl, wie 2-(N,N-Alkylenamino)propyl oder 2-(N,N-Alkylenamino)-2-methyl-propyl oder 3-(N,N-Alkylenamino)-C₃-C₇-alkyl, insbesondere 3-(N,N-Alkylenamino)-C₃-C₅-alkyl, wie 3-(N,N-Alkylenamino)propyl oder 3-(N,N-Alkylenamino)-2-methyl-propyl, wobei 4-bis 7-gliedriges N,N-Alkylenamino insbesondere Pyrrolidino, Piperidino oder in zweiter Linie Hexahydroazepino oder Octahydroazocino bedeutet.

2- oder 3-(Aza)niederalkylenaminoniederalkyl ist beispielsweise 4- bis 7-gliedriges 2-[N,N-(Aza)alkylenamino]-C₃-C₇-alkyl, insbesondere 2-[N,N-(Aza)alkylenamino]-C₃-C₅-alkyl, wie 2-[N,N-(Aza)alkylenamino]propyl oder 2-[N,N-(Aza)alkylenamino]-2-methyl-propyl oder 3-[N,N-(Aza)alkylenamino]-C₃-C₇-alkyl, insbesondere 3-[N,N-(Aza)alkylenamino]-C₃-C₅-alkyl, wie 3-[N,N-(Aza)alkylenamino]propyl oder 3-[N,N-(Aza)alkylenamino]-2-methyl-propyl, wobei 4-bis 7-gliedriges N,N-(Aza)alkylenamino insbesondere Piperazino oder N'-C₁-C₄-Alkyl-, wie N'-Methylpiperazino, oder N'-C₁-C₇-Alkanoyl-, wie N'-Acetyl- oder N' -Pivaloylpiperazino bedeutet.

2- oder 3-(Oxa)niederalkylenaminoniederalkyl ist beispielsweise 4- bis 7-gliedriges 2-[N,N-(Oxa)alkylenamino]-C₃-C₇-alkyl, insbesondere 2-[N,N-(Oxa)alkylenamino]-C₃-C₅-alkyl, wie 2-[N,N-(Oxa)alkylenamino]propyl oder 2-[N,N-(Oxa)alkylenamino]-2-methyl-propyl oder 3-[N,N-(Oxa)alkylenamino]-C₃-C₇-alkyl, insbesondere 3-[N,N-(Oxa)alkylenamino]-C₃-C₅-alkyl, wie 3-[N,N-(Oxa)alkylenamino]propyl oder 3-[N,N-(Oxa)alkylenamino]-2-methyl-propyl, wobei 4-bis 7-gliedriges N,N-(Oxa)alkylenamino insbesondere Morpholino bedeutet.

2- oder 3-(Thia)niederalkylenaminoniederalkyl ist beispielsweise 4- bis 7-gliedriges, gegebenenfalls S-oxidiertes 2-[N,N-(Thia)alkylenamino]-C₃-C₇-alkyl, insbesondere 2-[N,N-(Thia)alkylenamino]-C₃-C₅-alkyl, wie 2-[N,N-(Thia)alkylenamino]propyl oder 2-[N,N-(Thia)alkylenamino]-2-methyl-propyl oder 3-[N,N-(Thia)alkylenamino]-C₃-C₇-alkyl, insbesondere 3-[N,N-(Thia)alkylenamino]-C₃-C₅-alkyl, wie 3-[N,N-(Thia)alkylenamino]propyl oder 3-[N,N-(Thia)alkylenamino]-2-methyl-propyl, wobei 4-bis 7-gliedriges, gegebenenfalls S-oxidiertes N,N-(Thia)alkylenamino insbesondere Thiomorpholino oder S-Oxy- bzw. S,S-Dioxythiomorpholino bedeutet.

2- oder 3-Hydroxyniederalkyl ist beispielsweise 2-Hydroxy-C₃-C₇-alkyl, insbesondere Hydroxy-C₃-C₅-alkyl, wie 2-Hydroxypropyl oder 2-Hydroxy-2-methyl-propyl, oder 3-Hydroxy-C₃-C₇-alkyl, insbesondere 3-Hydroxy-C₃-C₅-alkyl, wie 3-Hydroxypropyl oder 3-Hydroxy-2-methyl-propyl.

2- oder 3-Niederalkanoyloxyniederalkyl ist beispielsweise 2-(C₁-C₇-Alkanoyloxy)-C₃-C₇-alkyl, insbesondere (C₁-C₇-Alkanoyloxy)-C₃-C₅-alkyl, wie 2-(C₁-C₇-Alkanoyloxy)propyl oder 2-(C₁-C₇-Alkanoyloxy)-2-methyl-propyl, oder 3-(C₁-C₇-Alkanoyloxy)-C₃-C₇-alkyl, insbesondere 3-(C₁-C₇-Alkanoyloxy)-C₃-C₅-alkyl, wie 3-(C₁-C₇-Alkanoyloxy)propyl oder 3-(C₁-C₇-Alkanoyloxy)-2-methyl-propyl, wobei C₁-C₇-Alkanoyloxy insbesondere C₁-C₄-Alkanoyloxy, wie Acetyloxy oder Pivaloyloxy, bedeutet.

2- oder 3-Niederalkoxycarbonyloxyniederalkyl ist beispielsweise 2-(C₁-C₇-Alkoxycarbonyloxy)-C₃-C₇-alkyl, insbesondere (C₁-C₇-Alkoxycarbonyloxy)-C₃-C₅-alkyl, wie 2-(C₁-C₇-Alkoxycarbonyloxy)propyl oder 2-(C₁-C₇-Alkoxycarbonyloxy)-2-methylpropyl, oder 3-(C₁-C₇-Alkoxycarbonyloxy)-C₃-C₇-alkyl, insbesondere 3-(C₁-C₇-Alkoxycarbonyloxy)-C₃-C₅-alkyl, wie 3-(C₁-C₇-Alkoxycarbonyloxy)propyl oder 3-(C₁-C₇-Alkoxycarbonyloxy)-2-methyl-propyl, wobei C₁-C₇-Alkoxycarbonyloxy insbesondere C₁-C₄-Alkoxycarbonyloxy, wie Tertiärbutyloxycarbonyloxy bedeutet.

2- oder 3-Triniederalkylsilyloxyniederalkyl ist beispielsweise 2-(Tri-C₁-C₇-alkylsilyloxy)-C₃-C₇-alkyl, insbesondere (Tri-C₁-C₇-alkylsilyloxy)-C₃-C₅-alkyl, wie 2-(Tri-C₁-C₇-alkylsilyloxy)propyl oder 2-(Tri-C₁-C₇-alkylsilyloxy)-2-methyl-propyl, oder 3-(Tri-C₁-C₇-alkylsilyloxy)-C₃-C₇-alkyl, insbesondere 3-(Tri-C₁-C₇-alkylsilyloxy)-C₃-C₅-alkyl, wie 3-(Tri-C₁-C₇-alkylsilyloxy)propyl oder 3-(Tri-C₁-C₇-alkylsilyloxy)-2-methyl-propyl, wobei Tri-C₁-C₇-alkylsilyloxy insbesondere Tri-C₁-C₄-alkylsilyloxy, wie Trimethylsilyloxy oder Tributylsilyloxy, oder C₄-C₇-Alkyl(di-C₁-C₄-alkyl)silyloxy, wie 1,2,2-Trimethylpropyl(dimethyl)silyloxy bedeutet.

Halogen ist beispielsweise Halogen der Atomnummer bis und mit 35, wie Chlor oder Fluor, ferner Brom.

2- oder 3-Haloniederalkyl ist beispielsweise 2-Halo-C₃-C₇-alkyl, insbesondere 2-Halo-C₃-C₅-alkyl, wie 2-Halopropyl oder 2-Halo-2-methyl-propyl oder 3-Halo-C₃-C₇-alkyl, insbesondere 3-Halo-C₃-C₅-alkyl, wie 3-Halopropyl oder 3-Halo-2-methyl-propyl, wobei Halo beispielsweise Halogen der Atomnummer bis und mit 35, wie Chlor oder Fluor, ferner Brom bedeutet.

Halomethyl ist beispielsweise Halomethyl, worin Halo die Atomnummer bis und mit 35 aufweist und beispielsweise Chlor oder Fluor, ferner Brom bedeutet.

Die Verbindungen der Formel I sind basisch und können Säureadditionssalze bilden. Pharmazeutisch verwendbare Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Fumarate, Maleate, Tartrate oder Citrate.

Die Verbindungen der Formel I und auf an sich bekannten Methoden beruhende Verfahren zu ihrer Herstellung sind bekannt und beispielsweise in GB-1,325,061, US-4,697,020, DE-2,405,395, DE-2,632,745, DE-2,632,746, DE-2,632,747, CH-594648, EP-085,275 und EP-22,515 als Zwischenprodukte für die Herstellung von tumorhemmenden Arzneimittelwirkstoffen beschrieben. Neue Verbindungen der Formel I und Ihre pharmazeutisch verwendbaren Salze können in analoger Weise hergestellt werden wie dort beschrieben. Ferner ist, beispielsweise aus DE-2,405,395 und CH-511,877, bekannt, dass Verbindungen der Formel I, worin R₁ Methyl oder Niederalk-2-en-1-yl, R₂ Wasserstoff, R₃ Methyl, R₄ eine Gruppe der Formeln Ia bzw. Id und R₅ Niederalk-2-en-1-yl bedeuten, ihrerseits tumorhemmende Eigenschaften aufweisen und sich insbesondere zur Behandlung von neoplastischen Erkrankungen bei Warmblütern eignen. Der Verbindung der Formel I, worin R₁ Allyl, R₂ Wasserstoff, R₃ Methyl, R₄ eine Gruppe der Formel Ia und R₅ Allyl darstellt, wurden in CH-511,877 bzw. BE-753,532 ferner nicht näher spezifizierte immunsuppresive Wirkungen zugeschrieben, auf Grund derer sich diese Verbindung für die Verbesserung der Überlebensfähigkeit von tranplantierten Organen sowie zur Behandlung von Autoimmunerkrankungen eignen soll.

Die vorliegende Erfindung beruht auf der überraschenden Feststellung, dass die Verbindungen der Formel I und ihre pharmazeutisch verwendbaren Salze zusätzlich zu ihrer bekannten tumorhemmenden Wirkung, die meist im täglichen Dosisbereich von etwa 10 bis etwa 250 mg/kg i.p. auftritt, bereits bei sehr geringer, noch nicht tumorhemmender Dosierung, ausgeprägte antiarthritische Eigenschaften aufweisen. Diese können *in vivo* beispielsweise am Modell der Adjuvans-Arthritis der Ratte nach I. Wiesenberg et al. Clin. Exp. Immunol. 78, 245 (1989) im Dosisbereich ab etwa 0,1 bis etwa 0,3 mg/kg p.o. oder i.p. nachgewiesen werden.

Der Mechanismus der antiarthritischen Wirkung der Verbindungen der Formel I und ihrer pharmazeutisch verwendbaren Salze ist derzeit noch nicht genau bekannt. Es kann jedoch ausgeschlossen werden, dass ein direkter immunsuppressiver Mechanismus via Lymphozytotoxizität oder Myelosuppression vorliegt, weil selbst Dosierungen, die mehr als 100-mal höher sind als die antiarthritisch wirksame Dosis, zu keiner sonst zu erwartenden massiven Suppression der lymphatischen Organe (Thymus, Milz) oder zu einer Leukopenie führen. Bei der Therapie rheumatoider Erkrankungen mit immunsuppressiv wirkenden Zytostatika, z.B. mit Cyclophosphamid, sind Lymphozytotoxizität und Myelosuppression bekannte unerwünschte Nebenwirkungen.

Hingegen konnte nachgewiesen werden, dass die Verbindungen der Formel I und ihre pharmazeutisch verwendbaren Salze in humanen Monozyten die Synthese des Zytokins Interleukin-1 hemmen. Interleukin-1 ist ein Entzündungsmediator, der in akuten und chronischen Entzündungsprozessen eine Schlüsselrolle spielt. Es ist deshalb davon auszugehen, dass die Verbindungen der Formel I und ihre pharmazeutisch verwendbaren Salze in Dosierungen, die niedriger sind als die zytostatisch wirksame Dosis, Eigenschaften besitzen, die die antiarthritischen Effekte bewirken. Ein ursächlicher Zusammenhang mit der genannten Zytokinsynthesehemmung könnte bestehen, obwohl auch andere, noch nicht bekannte immunmodulatorische Mechanismen nicht ausgeschlossen werden können.

Die Verbindungen der Formel I und ihre pharmazeutisch verwendbaren Salze können deshalb zur Behandlung von Erkrankungen des rheumatoiden Formenkreises im Sinne des "preliminary proposal of the Glossary Committee of the American Rheumatism Association" eingesetzt werden. Zu diesen gehören insbesondere die rheumatoide Arthritis, die juvenile Arthritis, die ankylosierende Spondylitis und andere seronegative Spondylathritiden, z.B. Spondylarthritis bei Colitis ulcerosa und Morbus Crohn, aber auch reaktive Arthritiden, Kollagenkrankheiten wie Lupus erythematosus, degenerative rheumatische Erkrankungen, extraartikuläre rheumatische und pararheumatische Erkrankungen, z.B. Gicht und Osteoporose.

Die Erfindung betrifft in erster Linie die Verwendung von Verbindungen der Formel I, worin R₁ Niederalk-2-en-1-yl, Niederalk-3-en-2-yl, Niederalk-2-in-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl bedeutet, R₂ Wasserstoff und R₃ Diniederalkylaminoniederalkyl oder Halomethyl bedeutet oder R₂ und R₃ unabhängig voneinander Wasserstoff, Niederalkyl oder gemeinsam Methylen darstellen, R₄ eine Gruppe der Formeln Ia, Ib oder Ic darstellt, in der R₅ Wasserstoff, Niederalkyl, Niederalk-3-in-2-yl oder Niederalk-2-in-1-yl und R₆ eine Gruppe der Formeln Id oder Ie mit den vorstehenden Bedeutungen von R₁, R₂, R₃ und R₅ bedeutet, und ihrer pharmazeutisch verwendbaren Salze zur Herstellung pharmazeutischer Präparate für die Behandlung von Erkrankungen des rheumatoiden Formenkreises.

Die Erfindung betrifft in vor allem die Verwendung von Verbindungen der Formel I, worin R₁ C₃-C₇-Alk-2-en-1-yl, wie Allyl oder Methallyl, C₃-C₇-Alk-3-en-2-yl, wie But-3-en-2-yl, C₃-C₇-Alk-2-in-1-yl, wie Prop-2-in-1-yl, oder 2-Halo-C₃-C₇-alkyl, wie 2-Bromisobutyl, bedeutet, R₂ Wasserstoff bedeutet und R₃ C₁-C₄-Alkyl, wie Methyl, Di-C₁-C₄-alkylaminomethyl, wie Dimethylaminomethyl, oder Halomethyl, wie Brommethyl, darstellt oder R₂ und R₃ unabhängig voneinander Wasserstoff oder C₁-C₄-Alkyl, wie Methyl, bedeuten oder gemeinsam für Methylen stehen, R₄ eine Gruppe der Formel Ia, Ib oder Ic darstellt, in der R₅ Wasserstoff, C₁-C₄-Alkyl, wie Methyl, C₃-C₇-Alk-2-en-1-yl, wie Allyl, oder C₃-C₇-Alk-2-in-1-yl, wie Prop-2-in-1-yl, bedeutet, R₆ eine Gruppe der Formeln Id oder Ie mit den vorstehenden Bedeutungen von R₁, R₂, R₃ und R₅ darstellt, und ihrer pharmazeutisch verwendbaren Salze zur Herstellung pharmazeutischer Präparate für die Behandlung von Erkrankungen des rheumatoiden Formenkreises.

Die Erfindung betrifft insbesondere die Verwendung von Verbindungen der Formel I, worin R₁ C₃-C₇-Alk-2-en-1-yl, wie Allyl oder Methallyl, C₃-C₇-Alk-2-in-1-yl, wie Prop-2-in-1-yl, oder 2-Halo-C₃-C₇-alkyl, wie 2-Bromisobutyl, bedeutet, R₂ und R₃ unabhängig voneinander Wasserstoff oder C₁-C₄-Alkyl, wie Methyl, bedeuten oder gemeinsam für Methylen stehen, R₄ eine Gruppe der Formel Ia, Ib oder Ic darstellt, in der R₅ C₁-C₄-Alkyl, wie Methyl, C₃-C₇-Alk-2-en-1-yl, wie Allyl, oder C₃-C₇-Alk-2-in-1-yl, wie Prop-2-in-1-yl, bedeutet und R₆ eine Gruppe der Formeln Id oder Ie mit den vorstehenden Bedeutungen von R₁, R₂, R₃ und R₅ darstellt, und ihrer pharmazeutisch verwendbaren Salze zur Herstellung pharmazeutischer Präparate für die Behandlung von Erkrankungen des rheumatoiden Formenkreises.

Die Erfindung betrifft in allererster Linie die Verwendung von Verbindungen der Formel I, worin R₁ C₃-C₇-Alk-2-en-1-yl, wie Allyl oder Methallyl, bedeutet, R₂ und R₃ unabhängig voneinander Wasserstoff oder C₁-C₄-Alkyl, wie Methyl, bedeuten, R₄ eine Gruppe der Formel Ia, Ib oder Ic darstellt, in der R₅ C₁-C₄-Alkyl, wie Methyl, oder C₃-C₇-Alk-2-en-1-yl, wie Allyl, bedeutet und R₆ eine Gruppe der Formeln Id oder Ie mit den vorstehenden Bedeutungen von R₁, R₂, R₃ und R₅ darstellt, und ihrer pharmazeutisch verwendbaren Salze zur Herstellung pharmazeutischer Präparate für die Behandlung von Erkrankungen des rheumatoiden Formenkreises.

Die Erfindung betrifft namentlich die Verwendung von
3-Prop-2-in-yl-thiazolidin-2,4-dion-2-(4-methyl-3-thiosemicarbazon);
3-Allyl-thiazolidin-2,4-dion-2-(4-allyl-3-thiosemicarbazon);
3-Allyl-thiazolidin-2,4-dion-2-(4-methyl-3-thiosemicarbazon);
3-Allyl-thiazolidin-2,4-dion-2-(4-propinyl-3-thiosemicarbazon);
3-Methallyl-5,5-dimethyl-thiazolidin-2,4-dion-2-(4-allyl-3-thiosemicarbazon);
3-Methallyl-thiazolidin-2,4-dion-2-(4-methyl-3-thiosemicarbazon);
3-Allyl-5-methyl-thiazolidin-2,4-dion-2-(4-allyl-3-thiosemicarbazon);
3-Methallyl-5-methyl-thiazolidin-2,4-dion-2-(4-methyl-3-thiosemicarbazon);
1,1'-Dithiobis-{N-methylformamid-[3-methallyl-4-oxo-5-methyl-thiazolidinyliden}-hydrazon;
1,1'-Dithiobis-{N-allylformamid-[3-allyl-4-oxo-5-methyl-thiazolidinyliden}-hydrazon;
1,1'-Dithiobis-{N-methylformamid-[3-methallyl-4-oxo-5,5-dimethyl-thiazolidinyliden}-hydrazon;
1,1'-Dithiobis-{N-allylformamid-[3-methallyl-4-oxo-5,5-dimethyl-thiazolidinyliden}-hydrazon und
1,1'-Dithiobis-{N-methylformamid-[3-allyl-4-oxo-thiazolidinyliden}-hydrazon
zur Herstellung pharmazeutischer Präparate für die Behandlung von Erkrankungen des rheumatoiden Formenkreises.

Bei den pharmazeutischen Präparaten, welche die Verbindung der Formel I oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die pharmazeutischen Präparate enthalten z.B. von etwa 10 % bis etwa 80 %, vorzugsweise von etwa 20 % bis etwa 60 %, des Wirkstoffs. Die pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyäthylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykol oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykol oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet oder wässrige Injektionssuspensionen, welche viskositatserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 5 mg bis etwa 1000 mg, insbesondere von etwa 10 mg bis etwa 200 mg zu veranschlagen. Diese kann auf einmal gegeben oder auf mehrere, z.B. 2 bis 4 Einzeldosen verteilt werden. Pharmazeutische Präparate in Dosiseinheitsform enthalten somit von etwa 5 mg bis etwa 250 mg, insbesondere von etwa 10 mg bis etwa 50 mg.

Die nachfolgen Beispiele dienen zur Illustration der Erfindung.

### Beispiel 1: Tabletten, enthaltend je 10 mg 3-Allyl-5-methyl-thiazolidin-2,4-dion-2-(4-allyl-3-thiosemicarbazon)oder ein Salz, z.B. das Diathanolammoniumsalz, davon können wie folgt hergestellt werden:

### Zusammensetzung (10000 Tabletten)

| | |
|---|---|
| Wirkstoff | 100,0 g |
| Lactose | 450,0 g |
| Kartoffelstärke | 350,0 g |
| Gelatine | 10,0 g |
| Talk | 60,0 g |
| Magnesiumstearat | 10,0 g |
| Siliciumdioxid (hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 100,0 mg Gewicht und 10,0 mg Wirkstoffgehalt, die gewünschtenfalls mit Teilkerben zur feineren Anpassung der Dosierung versehen sein können.

### Beispiel 2: Gelatinesteckkapseln, enthaltend 20 mg Wirkstoff, z.B. 3-Allyl-5-methylthiazolidin-2,4-dion-2-(4-allyl-3-thiosemicarbazon)oder ein Salz, z.B. das Diäthanolammoniumsalz, davon können z.B. folgendermassen hergestellt werden:

### Zusammensetzung (für 1000 Kapseln)

| | |
|---|---|
| Wirkstoff | 20,0 g |
| Lactose | 240,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3-minütigem weiteren Mischen werden je 300 mg der erhaltenen Formulierung in Gelatinesteckkapseln der Grösse 0 abgefüllt.

### Beispiel 3: Gelatinesteckkapseln, enthaltend 100 mg Wirkstoff, z.B. 3-Allyl-5-methylthiazolidin-2,4-dion-2-(4-allyl-3-thiosemicarbazon)oder ein Salz, z.B. das Diäthanolammoniumsalz, davon können z.B. folgendermassen hergestellt werden:

### Zusammensetzung (für 1000 Kapseln)

| | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

### Beispiel 4: Lacktabletten, enthaltend je 50 mg 3-Allyl-5-methyl-thiazolidin-2,4-dion-2-(4-allyl-3-thiosemicarbazon)oder ein Salz, z.B. das Diäthanolammoniumsalz, davon können wie folgt hergestellt werden:

### Zusammensetzung (für 1000 Lacktabletten)

| | |
|---|---|
| Wirkstoff | 50,0 g |
| Lactose | 100,0 g |
| Maisstärke | 70,0 g |
| Talk | 10,0 g |
| Calciumstearat | 2,0 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstarke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 232 mg) verpresst und diese mit einer Lösung der Hydroxypropyl-methylcellulose und des Schellacks in Methylenchlorid lackiert; Endgewicht der Lacktablette: 235 mg.

### Beispiel 5: Eine 0,2%-ige Injektions- oder Infusionslösung von 3-Allyl-5-methylthiazolidin-2,4-dion-2-(4-allyl-3-thiosemicarbazon)oder ein Salz, z.B. das Diäthanolammoniumsalz, davon kann beispielsweise folgendermassen hergestellt werden:

### Zusammensetzung (für 1000 Ampullen)

| | |
|---|---|
| Wirkstoff | 5,0 g |
| Natriumchlorid | 22,5 g |
| Phosphatpuffer pH=7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

### Beispiel 6: 1%-ige Salbe (O/W-Emulsion), als Wirkstoff z.B. 3-Allyl-5-methylthiazolidin-2,4-dion-2-(4-allyl-3-thiosemicarbazon)oder ein Salz, z.B. das Diäthanolammoniumsalz, davon enthaltend, mit folgender Zusammensetzung:

| | |
|---|---|
| Wirkstoff | 1,0 g |
| Cetylalkohol | 3,0 g |
| Glycerin | 6,0 g |
| Methylparaben® | 0,18 g |
| Propylparaben® | 0,05 g |
| Arlacel® 60 | 0,6 g |
| Tween® 60 | 4,4 g |
| Stearinsäure | 9,0 g |
| Isopropylpalmitat | 2,0 g |
| Paraffinöl dickflüssig | 10,0 g |
| Wasser entmin. q.s. ad | 100,0 g |

### Beispiel 7: 1%-iges Gel, als Wirkstoff z.B. 3-Allyl-5-methyl-thiazolidin-2,4-dion-2-(4-allyl-3-thiosemicarbazon)oder ein Salz, z.B. das Diäthanolammoniumsalz, davon enthaltend, mit folgender Zusammensetzung:

| | |
|---|---|
| Wirkstoff | 1,0 g |
| Carbopol® 934 P | 1,0 g |
| Glycerin | 3,0 g |
| Isopropanol | 25,0 g |
| Softigen® 767 | 0,2 g |
| Wasser entmin. q.s. ad | 100,0 g |

### Beispiel 8: In analoger Weise wie in den vorstehenden Beispielen 1 bis 7 beschrieben kann man auch pharmazeutische Präparate, enthaltend eine andere Verbindung der Formel I, beispielsweise

3-Prop-2-in-yl-thiazolidin-2,4-dion-2-(4-methyl-3-thiosemicarbazon);
3-Allyl-thiazolidin-2,4-dion-2-(4-allyl-3-thiosemicarbazon);
3-Allyl-thiazolidin-2,4-dion-2-(4-methyl-3-thiosemicarbazon);
3-Allyl-thiazolidin-2,4-dion-2-(4-propinyl-3-thiosemicarbazon);
3-Methallyl-5,5-dimethyl-thiazolidin-2,4-dion-2-(4-allyl-3-thiosemicarbazon);
3-Methallyl-thiazolidin-2,4-dion-2-(4-methyl-3-thiosemicarbazon);
3-Methallyl-5-methyl-thiazolidin-2,4-dion-2-(4-methyl-3-thiosemicarbazon);
3-Propyl-thiazolidin-2,4-dion-2-(4-methyl-3-thiosemicarbazon);
1,1'-Dithiobis-{N-methylformamid-[3-methallyl-4-oxo-5-methyl-thiazolidinyliden}-hydrazon;
1,1'-Dithiobis-{N-allylformamid-[3-methallyl-4-oxo-5-methyl-thiazolidinyliden}-hydrazon;
1,1'-Dithiobis-{N-methylformamid-[3-methallyl-4-oxo-5,5-dimethyl-thiazolidinyliden}-hydrazon;
1,1'-Dithiobis-{N-allylformamid-[3-methallyl-4-oxo-5,5-dimethyl-thiazolidinyliden}-hydrazon oder
1,1'-Dithiobis-{N-methylformamid-[3-allyl-4-oxo-thiazolidinyliden}-hydrazon
oder ein pharmazeutisch verwendbares Salz davon herstellen.

## Patentansprüche

1. Verwendung von Verbindungen der Formel I 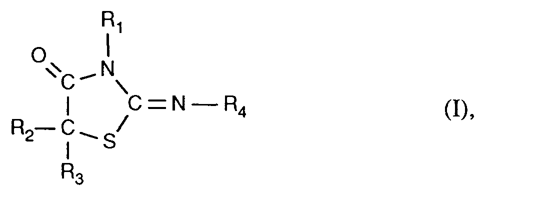 worin R₁ Niederalk-2-en-1-yl, Niederalk-3-en-2-yl, Niederalk-2-in-1-yl oder in 2,3-Stellung durch unter Bildung einer Doppelbindung eliminierbare Reste substituiertes Niederalkyl bedeutet, R₂ Wasserstoff und R₃ unsubstituiertes oder durch zusammen mit Wasserstoff R₂ unter Bildung einer Doppelbindung eliminierbare Reste substituiertes Methyl bedeutet oder R₂ und R₃ beide Wasserstoff oder Niederalkyl bedeuten oder gemeinsam Methylen darstellen und R₄ eine Gruppe der Formeln Ia, Ib oder Ic 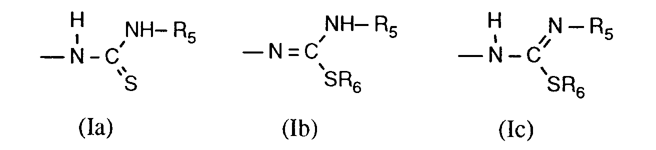 darstellt, in der R₅ Wasserstoff, Niederalkyl, Niederalk-2-en-1-yl oder Niederalk-2-in-1-yl und R₆ eine Gruppe der Formel Id oder Ie 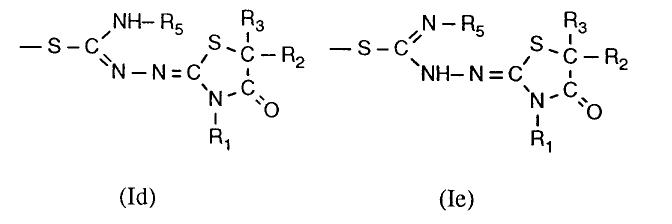 bedeutet, in denen R₁, R₂, R₃ und R₅ die angegebenen Bedeutungen haben, oder ein pharmazeutisch verwendbares Salz davon neben üblichen pharmazeutischen Hilfsstoffen zur Herstellung pharmazeutischer Präparate für die Behandlung von Erkrankungen des rheumatoiden Formenkreises.

2. Verwendung gemäss Anspruch 1 zur Herstellung pharmazeutischer Präparate für die Behandlung von Erkrankungen des rheumatoiden Formenkreises zur enteralen bzw. parenteralen Verabreichung.

3. Verwendung gemäss Anspruch 1 zur Herstellung pharmazeutischer Präparate in Dosiseinheitsform, wie Dragées, Tabletten, Kapseln oder Suppositorien, ferner Injektionslösungen oder Infusionslösungen.

4. Verwendung gemäss Anspruch 1 zur Herstellung pharmazeutischer Präparate gemäss einem der Ansprüche 1 bis 3 enthaltend von etwa 5 mg bis etwa 250 mg einer Verbindung der Formel I oder eines pharmazeutisch verwendbaren Salzes davon.

5. Verwendung gemäss Anspruch 1 zur Herstellung pharmazeutischer Präparate gemäss einem der Ansprüche 1 bis 3 enthaltend von etwa 10 mg bis etwa 50 mg einer Verbindung der Formel I oder eines pharmazeutisch verwendbaren Salzes davon.

6. Verwendung gemäss einen, der Ansprüche 1 bis 5, dadurch gekennzeichnet, dass man eine Verbindung der Formel I, worin R₁ Niederalk-2-en-1-yl, Niederalk-3-en-2-yl, Niederalk-2-in-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl bedeutet, R₂ Wasserstoff und R₃ Diniederalkylaminoniederalkyl oder Halomethyl bedeutet oder R₂ und R₃ unabhängig voneinander Wasserstoff, Niederalkyl oder gemeinsam Methylen darstellen, R₄ eine Gruppe der Formeln Ia, Ib oder Ic darstellt, in der R₅ Wasserstoff, Niederalkyl, Niederalk-3-in-2-yl oder Niederalk-2-in-1-yl und R₆ eine Gruppe der Formeln Id oder Ie mit den vorstehenden Bedeutungen von R₁, R₂, R₃ und R₅ bedeutet, oder ein pharmazeutisch verwendbares Salz davon wählt.

7. Verwendung gemäss einem der Ansprüche 1 bis 5, dadurch gekennzeichnet, dass man eine Verbindung der Formel I, worin R₁ C₃-C₇-Alk-2-en-1-yl, C₃-C₇-Alk-3-en-2-yl, C₃-C₇-Alk-2-in-1-yl oder 2-Halo-C₃-C₇-alkyl bedeutet, R₂ Wasserstoff bedeutet und R₃ C₁-C₄-Alkyl, Di-C₁-C₄-alkylaminomethyl, oder Halomethyl darstellt oder R₂ und R₃ unabhängig voneinander Wasserstoff oder C₁-C₄-Alkyl bedeuten oder gemeinsam für Methylen stehen, R₄ eine Gruppe der Formel Ia, Ib oder Ic darstellt, in der R₅ Wasserstoff, C₁-C₄-Alkyl, C₃-C₇-Alk-2-en-1-yl oder C₃-C₇-Alk-2-in-1-yl bedeutet und R₆ eine Gruppe der Formeln Id oder Ie mit den vorstehenden Bedeutungen von R₁, R₂, R₃ und R₅ darstellt, oder ein pharmazeutisch verwendbares Salz davon wählt.

8. Verwendung gemäss einem der Ansprüche 1 bis 5, dadurch gekennzeichnet, dass man eine Verbindung der Formel I, worin R₁ C₃-C₇-Alk-2-en-1-yl, wie Allyl oder Methallyl, C₃-C₇-Alk-2-in-1-yl, wie Prop-2-in-1-yl, oder 2-Halo-C₃-C₇-alkyl, wie 2-Bromisobutyl, bedeutet, R₂ und R₃ unabhängig voneinander Wasserstoff oder C₁-C₄-Alkyl, wie Methyl, bedeuten oder gemeinsam für Methylen stehen, R₄ eine Gruppe der Formel Ia, Ib oder Ic darstellt, in der R₅ C₁-C₄-Alkyl, wie Methyl, C₃-C₇-Alk-2-en-1-yl, wie Allyl, oder C₃-C₇-Alk-2-in-1-yl, wie Prop-2-in-1-yl, bedeutet und R₆ eine Gruppe der Formeln Id oder Ie mit den vorstehenden Bedeutungen von R₁, R₂, R₃ und R₅ darstellt, oder ein pharmazeutisch verwendbares Salz davon wählt.

9. Verwendung gemäss einem der Ansprüche 1 bis 5, dadurch gekennzeichnet, dass man eine Verbindung der Formel I, worin R₁ C₃-C₇-Alk-2-en-1-yl, wie Allyl oder Methallyl, bedeutet, R₂ und R₃ unabhängig voneinander Wasserstoff oder C₁-C₄-Alkyl, wie Methyl, bedeuten, R₄ eine Gruppe der Formel Ia, Ib oder Ic darstellt, in der R₅ C₁-C₄-Alkyl, wie Methyl, oderC₃-C₇-Alk-2-en-1-yl, wie Allyl, bedeutet, R₆ eine Gruppe der Formeln Id oder Ie mit den vorstehenden Bedeutungen von R₁, R₂, R₃ und R₅ darstellt, oder ein pharmazeutisch verwendbares Salz davon wählt.

10. Verwendung gemäss einem der Ansprüche 1 bis 5, dadurch gekennzeichnet, dass man als Verbindung der Formel I 3-Allyl-thiazolidin-2,4-dion-2-(4-methyl-3-thiosemicarbazon) oder ein pharmazeutisch verwendbares Salz davon wählt.

11. Verwendung gemäss einem der Ansprüche 1 bis 5, dadurch gekennzeichnet, dass man als Verbindung der Formel I 3-Methallyl-5,5-dimethyl-thiazolidin-2,4-dion-2-(4-allyl-3-thiosemicarbazon) oder ein pharmazeutisch verwendbares Salz davon wählt.

12. Verwendung gemäss einem der Ansprüche 1 bis 5, dadurch gekennzeichnet, dass man als Verbindung der Formel I 3-Methallyl-thiazolidin-2,4-dion-2-(4-methyl-3-thiosemicarbazon) oder ein pharmazeutisch verwendbares Salz davon wählt.

13. Verwendung gemäss einem der Ansprüche 1 bis 5, dadurch gekennzeichnet, dass man als Verbindung der Formel I 3-Allyl-5-methyl-thiazolidin-2,4-dion-2-(4-allyl-3-thiosemicarbazon) oder ein pharmazeutisch verwendbares Salz davon wählt.

14. Verwendung gemäss einem der Ansprüche 1 bis 5, dadurch gekennzeichnet, dass man als Verbindung der Formel I 3-Methallyl-5-methyl-thiazolidin-2,4-dion-2-(4-methyl-3-thiosemicarbazon) oder ein pharmazeutisch verwendbares Salz davon wählt.

15. Verwendung gemäss einem der Ansprüche 1 bis 5, dadurch gekennzeichnet, dass man als Verbindung der Formel I 3-Propyl-thiazolidin-2,4-dion-2-(4-methyl-3-thiosemicarbazon) oder ein pharmazeutisch verwendbares Salz davon wählt.

16. Verwendung gemäss einem der Ansprüche 1 bis 5, dadurch gekennzeichnet, dass man als Verbindung der Formel I 3-Prop-2-in-yl-thiazolidin-2,4-dion-2-(4-methyl-3-thiosemicarbazon) oder ein pharmazeutisch verwendbares Salz davon wählt.

17. Verwendung gemäss einem der Ansprüche 1 bis 5, dadurch gekennzeichnet, dass man als Verbindung der Formel I 1,1'-Dithiobis-{N-methylformamid-[3-methallyl-4-oxo-5-methyl-thiazolidinyliden}-hydrazon oder ein pharmazeutisch verwendbares Salz davon wählt.

18. Verwendung gemäss einem der Ansprüche 1 bis 5, dadurch gekennzeichnet, dass man als Verbindung der Formel I 1,1'-Dithiobis-{N-allylformamid-[3-allyl-4-oxo-5-methyl-thiazolidinyliden}-hydrazon oder ein pharmazeutisch verwendbares Salz davon wählt.

19. Verwendung gemäss einem der Ansprüche 1 bis 5, dadurch gekennzeichnet, dass man als Verbindung der Formel I 1,1'-Dithiobis-{N-methylformamid-[3-methallyl-4-oxo-5,5-dimethyl-thiazolidinyliden}-hydrazon oder ein pharmazeutisch verwendbares Salz davon wählt.

20. Verwendung gemäss einem der Ansprüche 1 bis 5, dadurch gekennzeichnet, dass man als Verbindung der Formel I 1,1'-Dithiobis-{N-allylformamid-[3-methallyl-4-oxo-5,5-dimethyl-thiazolidinyliden}-hydrazon oder ein pharmazeutisch verwendbares Salz davon wählt.

21. Verwendung gemäss einem der Ansprüche 1 bis 5, dadurch gekennzeichnet, dass man als Verbindung der Formel I 1,1'-Dithiobis-{N-methylformamid-[3-allyl-4-oxo-thiazolidinyliden}-hydrazon oder ein pharmazeutisch verwendbares Salz davon wählt.

22. Verwendung gemäss einem der Ansprüche 1 bis 5, dadurch gekennzeichnet, dass man als Verbindung der Formel I 3-Allyl-thiazolidin-2,4-dion-2-(4-allyl-3-thiosemicarbazon, oder ein pharmazeutisch verwendbares Salz davon wählt.

23. Verwendung gemäss einem der Ansprüche 1 bis 5, dadurch gekennzeichnet, dass man als Verbindung der Formel I 3-Allyl-thiazolidin-2,4-dion-2-(4-propinyl-3-thiosemicarbazon, oder ein pharmazeutisch verwendbares Salz davon wählt.

## Claims

1. The use of a compound of the formula I 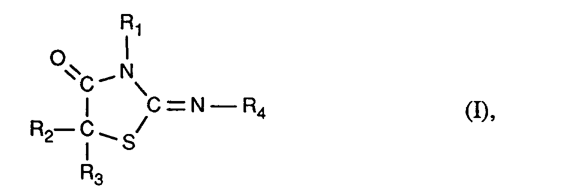 in which R₁ is lower alk-2-en-1-yl, lower alk-3-en-2-yl, lower alk-2-yn-1-yl or lower alkyl substituted in the 2,3-position by radicals which can be eliminated to give a double bond, R₂ is hydrogen and R₃ is methyl which is unsubstituted or substituted by radicals which can be eliminated together with hydrogen R₂ to give a double bond or R₂ and R₃ are both hydrogen or lower alkyl or are together methylene and R₄ is a group of the formula Ia, Ib or Ic 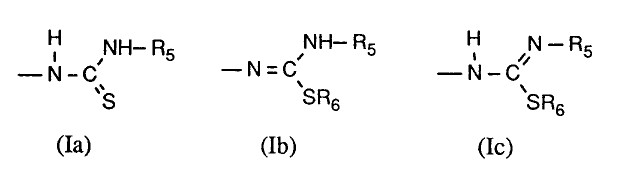 in which R₅ is hydrogen, lower alkyl, lower alk-2-en-1-yl or lower alk-2-yn-1-yl and R₆ is a group of the formula Id or Ie 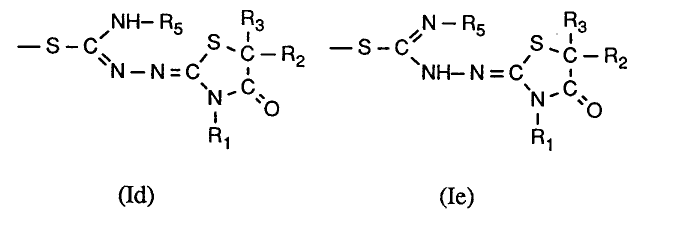 in which R₁, R₂, R₃ and R₅ are as defined, or a pharmaceutically acceptable salt thereof in addition to customary pharmaceutical adjuncts for the treatment of diseases of the rheumatoid type.

2. The use according to claim 1 for the production of a pharmaceutical preparation for the treatment of diseases of the rheumatoid type for enteral or parenteral administration.

3. The use according to claim 1 for the production of a pharmaceutical preparation in unit dose form, as sugar coated tablets, tablets, capsules or suppositories, and also injection or infusion solutions.

4. The use according to claim 1 for the production of a pharmaceutical preparation comprising from about 5 mg to about 250 mg of a compound of the formula I or a pharmaceutically acceptable salt thereof.

5. The use according to claim 1 for the production of a pharmaceutical preparation comprising from about 10 mg to about 50 mg of a compound of the formula I or a pharmaceutically acceptable salt thereof.

6. The use according to any one of claims 1 to 5, wherein a compound of the formula I in which R₁ is lower alk-2-en-1-yl, lower alk-3-en-2-yl, lower alk-2-yn-1-yl, 2- or 3-amino-, 2- or 3-lower alkylamino- or 2- or 3-di-lower alkylamino-lower alkyl, 2- or 3-lower alkyleneamino-, 2- or 3-(aza)lower alkyleneamino-, 2- or 3-(oxa)lower alkyleneamino- or 2- or 3-(thia)lower alkyleneamino-lower alkyl, 2- or 3-hydroxy-, 2- or 3-lower alkanoyloxy-, 2- or 3-lower alkoxycarbonyloxy- or 2- or 3-tri-lower alkylsilyloxy-lower alkyl or 2- or 3-halo-lower alkyl, R₂ is hydrogen and R₃ is di-lower alkylamino-lower alkyl or halomethyl or R₂ and R₃, independently of one another, are hydrogen or lower alkyl or together are methylene, R₄ is a group of the formula Ia, Ib or Ic in which R₅ is hydrogen, lower alkyl, lower alk-3-yn-2-yl or lower alk-2-yn-1-yl and R₆ is a group of the formula Id or Ie with R₁, R₂, R₃ and R₅ as defined above or a pharmaceutically acceptable salt thereof is chosen.

7. The use according to any one of claims 1 to 5, wherein a compound of the formula I in which R₁ is C₃-C₇alk-2-en-1-yl, C₃-C₇alk-3-en-2-yl, C₃-C₇alk-2-yn-1-yl or 2-halo-C₃-C₇alkyl, R₂ is hydrogen and R₃ is C₁-C₄alkyl, di-C₁-C₄alkylaminomethyl or halomethyl, or R₂ and R₃, independently of one another, are hydrogen or C₁-C₄alkyl or together are methylene, R₄ is a group of the formula Ia, Ib or Ic in which R₅ is hydrogen, C₁-C₄alkyl, C₃-C₇alk-2-en-1-yl or C₃-C₇alk-2-yn-1-yl, and R₆ is a group of the formula Id or Ie with R₁, R₂, R₃ and R₅ as defined above or a pharmaceutically acceptable salt thereof is chosen.

8. The use according to any one of claims 1 to 5, wherein a compound of the formula I in which R₁ is C₃-C₇alk-2-en-1-yl, such as allyl or methallyl, C₃-C₇alk-2-yn-1-yl, such as prop-2-yn-1-yl, or 2-halo-C₃-C₇alkyl, such as 2-bromoisobutyl, R₂ and R₃, independently of one another, are hydrogen or C₁-C₄alkyl, such as methyl, or together are methylene, R₄ is a group of the formula Ia, Ib or Ic in which R₅ is C₁-C₄alkyl, such as methyl, C₃-C₇alk-2-en-1-yl, such as allyl, or C₃-C₇alk-2-yn-1-yl, such as prop-2-yn-1-yl, and R₆ is a group of the formula Id or Ie with R₁, R₂, R₃ and R₅ as defined above or a pharmaceutically acceptable salt thereof is chosen.

9. The use according to any one of claims 1 to 5, wherein a compound of the formula I in which R₁ is C₃-C₇alk-2-en-1-yl, such as allyl or methallyl, R₂ and R₃, independently of one another, are hydrogen or C₁-C₄alkyl, such as methyl, R₄ is a group of the formula Ia, Ib or Ic in which R₅ is C₁-C₄alkyl, such as methyl, or C₃-C₇alk-2-en-1-yl, such as allyl, and R₆ is a group of the formula Id or Ie with R₁, R₂, R₃ and R₅ as defined above or a pharmaceutically acceptable salt thereof is chosen.

10. The use according to any one of claims 1 to 5, wherein the compound of the formula I chosen is 3-allylthiazolidine-2,4-dione-2-(4-methyl-3-thiosemicarbazone) or a pharmaceutically acceptable salt thereof.

11. The use according to any one of claims 1 to 5, wherein the compound of the formula I chosen is 3-methallyl-5,5-dimethylthiazolidine-2,4-dione-2-(4-allyl-3-thiosemicarbazone) or a pharmaceutically acceptable salt thereof.

12. The use according to any one of claims 1 to 5, wherein the compound of the formula I chosen is 3-methallylthiazolidine-2,4-dione-2-(4-methyl-3-thiosemicarbazone) or a pharmaceutically acceptable salt thereof.

13. The use according to any one of claims 1 to 5, wherein the compound of the formula I chosen is 3-allyl-5-methylthiazolidine-2,4-dione-2-(4-allyl-3-thiosemicarbazone) or a pharmaceutically acceptable salt thereof.

14. The use according to any one of claims 1 to 5, wherein the compound of the formula I chosen is 3-methallyl-5-methylthiazolidine-2,4-dione-2-(4-methyl-3-thiosemicarbazone) or a pharmaceutically acceptable salt thereof.

15. The use according to any one of claims 1 to 5, wherein the compound of the formula I chosen is 3-propylthiazolidine-2,4-dione-2-(4-methyl-3-thiosemicarbazone) or a pharmaceutically acceptable salt thereof.

16. The use according to any one of claims 1 to 5, wherein the compound of the formula I chosen is 3-prop-2-ynylthiazolidine-2,4-dione-2-(4-methyl-3-thiosemicarbazone) or a pharmaceutically acceptable salt thereof.

17. The use according to any one of claims 1 to 5, wherein the compound of the formula I chosen is 1,1'-dithiobis{N-methylformamide [3-methallyl-4-oxo-5-methylthiazolidinylidene]}hydrazone or a pharmaceutically acceptable salt thereof.

18. The use according to any one of claims 1 to 5, wherein the compound of the formula I chosen is 1,1'-dithiobis{N-allylformamide [3-allyl-4-oxo-5-methylthiazolidinylidene]}hydrazone or a pharmaceutically acceptable salt thereof.

19. The use according to any one of claims 1 to 5, wherein the compound of the formula I chosen is 1,1'-dithiobis{N-methylformamide [3-methallyl-4-oxo-5,5-dimethylthiazolidinylidene]}hydrazone or a pharmaceutically acceptable salt thereof.

20. The use according to any one of claims 1 to 5, wherein the compound of the formula I chosen is 1,1'-dithiobis{N-allylformamide [3-methallyl-4-oxo-5,5-dimethylthiazolidinylidene]}hydrazone or a pharmaceutically acceptable salt thereof.

21. The use according to any one of claims 1 to 5, wherein the compound of the formula I chosen is 1,1'-dithiobis{N-methylformamide [3-allyl-4-oxothiazolidinylidene]}hydrazone or a pharmaceutically acceptable salt thereof.

22. The use according to any one of claims 1 to 5, wherein the compound of the formula I chosen is 3-allylthiazolidine-2,4-dione-2-(4-allyl-3-thiosemicarbazone) or a pharmaceutically acceptable salt thereof.

23. The use according to any one of claims 1 to 5, wherein the compound of the formula I chosen is 3-allylthiazolidine-2,4-dione-2-(4-propynyl-3-thiosemicarbazone) or a pharmaceutically acceptable salt thereof.

## Revendications

1. Utilisation des composés de formule I 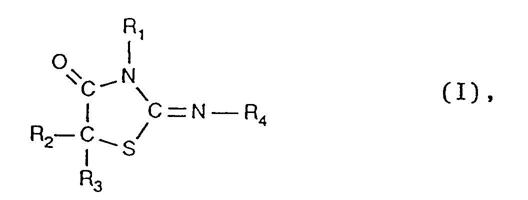 où R₁ représente un alc-2-én-1-yle inférieur, un alc-3-én-2-yle inférieur, un alc-2-yn-1-yle inférieur ou un alkyle inférieur substitué en position 2,3 par des restes éliminables en formant une double liaison, R₂ l'hydrogène et R₃ le méthyle non substitué ou substitué par des restes éliminables avec l'hydrogène R₂ en formant une double liaison ou R₂ et R₃ représentent tous les deux l'hydrogène ou un alkyle inférieur ou représentent ensemble le méthylène et R₄ un groupe de formule Ia, Ib ou Ic 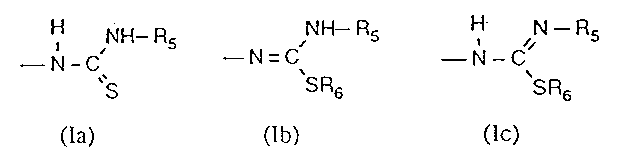 dans lesquelles R₅ représente l'hydrogène, un alkyle inférieur, un alc-2-én-1-yle inférieur ou un alc-2-yn-1-yle inférieur et R₆ un groupe de formule Id ou Ie 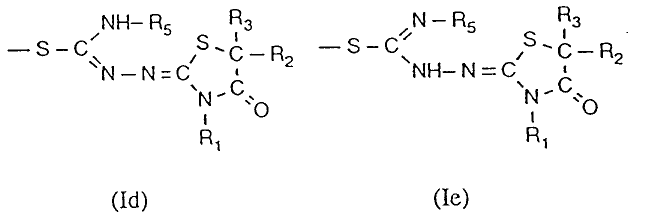 dans lesquelles R₁, R₂, R₃ et R₅ ont les significations indiquées ou d'un de leurs sels pharmaceutiquement utilisables, à côté des adjuvants pharmaceutiques usuels pour la préparation des compositions pharmaceutiques destinées au traitement des maladies des différentes formes rhumatoïdes.

2. Utilisation selon la revendication 1 pour la préparation de compositions pharmaceutiques pour le traitement des maladies des différentes formes rhumatoïdes destinées à l'administration entérale ou parentérale.

3. Utilisation selon la revendication 1 pour la préparation de compositions pharmaceutiques sous forme de doses unitaires telles que dragées, comprimés, capsules ou suppositoires, également de solutions pour injection ou de solutions pour perfusion.

4. Utilisation selon la revendication 1 pour la préparation de compositions pharmaceutiques selon l'une des revendications 1 à 3 contenant environ 5 mg à environ 250 mg d'un composé de formule I ou de l'un de ses sels pharmaceutiquement utilisables.

5. Utilisation selon la revendication 1 pour la préparation de compositions pharmaceutiques selon l'une des revendications 1 à 3 contenant environ 10 mg à environ 50 mg d'un composé de formule I ou de l'un de ses sels pharmaceutiquement utilisables.

6. Utilisation selon l'une des revendications 1 à 5, caractérisée en ce que l'on choisit un composé de formule I où R₁ représente un alc-2-én-1-yle inférieur, un alc-3-én-2-yle inférieur, un alc-2-yn-1-yle inférieur, un 2- ou 3-aminoalkyle inférieur, un 2- ou 3-alkyle inférieur aminoalkyle inférieur ou un 2- ou 3-dialkyle inférieur aminoalkyle inférieur, un 2- ou 3-alkylène inférieur aminoalkyle inférieur, un 2- ou 3-(aza)alkylène inférieur aminoalkyle inférieur, un 2- ou 3-(oxa)alkylène inférieur aminoalkyle inférieur ou un 2- ou 3-(thia)alkylène inférieur aminoalkyle inférieur, un 2- ou 3-hydroxyalkyle inférieur, un 2- ou 3-alcanoyloxy inférieur alkyle inférieur, un 2- ou 3-alcoxy inférieur carbonyloxyalkyle inférieur ou un 2- ou 3-trialkyle inférieur silyloxyalkyle inférieur ou un 2- ou 3-haloalkyle inférieur, R₂ représente l'hydrogène et R₃ un dialkyle inférieur aminoalkyle inférieur ou un halométhyle ou R₂ et R₃ représentent, indépendamment l'un de l'autre, l'hydrogène, un alkyle inférieur ou représentent ensemble le méthylène, R₄ représente un groupe de formule Ia, Ib ou Ic dans lesquelles R₅ représente l'hydrogène, un alkyle inférieur, un alc-3-yn-2-yle inférieur ou un alc-2-yn-1-yle inférieur et R₆ représente un groupe de formule Id ou Ie, R₁, R₂, R₃ et R₅ ayant les significations précédentes, ou l'un de ses sels pharmaceutiquement utilisables.

7. Utilisation selon l'une des revendications 1 à 5, caractérisée en ce que l'on choisit un composé de formule I où R₁ représente un alc-2-én-1-yle en C₃-C₇, un alc-3-én-2-yle en C₃-C₇, un alc-2-yn-1-yle en C₃-C₇ ou un 2-haloalkyle en C₃-C₇, R₂ représente l'hydrogène et R₃ un alkyle en C₁-C₄, un dialkyle en C₁-C₄ aminométhyle ou un halométhyle ou R₂ et R₃ représentent, indépendamment l'un de l'autre, l'hydrogène ou un alkyle en C₁-C₄ ou représentent ensemble le méthylène, R₄ représente un groupe de formule Ia, Ib ou Ic dans lesquelles R₅ représente l'hydrogène, un alkyle en C₁-C₄, un alc-2-én-1-yle en C₃-C₇ ou un alc-2-yn-1-yle en C₃-C₇ et R₆ représente un groupe de formule Id ou Ie, R₁, R₂, R₃ et R₅ ayant les significations précédentes ou l'un de ses sels pharmaceutiquement utilisables.

8. Utilisation selon l'une des revendications 1 à 5, caractérisée en ce que l'on choisit un composé de formule I où R₁ représente un alc-2-én-1-yle en C₃-C₇ tel que l'allyle ou le méthallyle, un alc-2-yn-1-yle en C₃-C₇ tel que le prop-2-yn-1-yle ou un 2-haloalkyle en C₃-C₇ tel que le 2-bromoisobutyle, R₂ et R₃ représentent, indépendamment l'un de l'autre, l'hydrogène ou un alkyle en C₁-C₄ tel que le méthyle ou représentent ensemble le méthylène, R₄ représente un groupe de formule Ia, Ib ou Ic dans lesquelles R₅ représente un alkyle en C₁-C₄ tel que le méthyle, un alc-2-én-1-yle en C₃-C₇ tel que l'allyle ou un alc-2-yn-1-yle en C₃-C₇ tel que le prop-2-yn-1-yle et R₆ représente un groupe de formule Id ou Ie, R₁, R₂, R₃ et R₅ ayant les significations précédentes ou l'un de ses sels pharmaceutiquement utilisables.

9. Utilisation selon l'une des revendications 1 à 5, caractérisée en ce que l'on choisit un composé de formule I où R₁ représente un alc-2-én-1-yle en C₃-C₇ tel que l'allyle ou le méthallyle, R₂ et R₃ représentent, indépendamment l'un de l'autre, l'hydrogène ou un alkyle en C₁-C₄ tel que le méthyle, R₄ représente un groupe de formule Ia, Ib ou Ic dans lesquelles R₅ représente un alkyle en C₁-C₄ tel que le méthyle ou un alc-2-én-1-yle en C₃-C₇ tel que l'allyle et R₆ un groupe de formule Id ou Ie, R₁, R₂, R₃ et R₅ ayant les significations précédentes ou l'un de ses sels pharmaceutiquement utilisables.

10. Utilisation selon l'une des revendications 1 à 5, caractérisée en ce que l'on choisit comme composé de formule I la 3-allyl-thiazolidine-2,4-dione-2-(4-méthyl-3-thiosemicarbazone) ou l'un de ses sels pharmaceutiquement utilisables.

11. Utilisation selon l'une des revendications 1 à 5, caractérisée en ce que l'on choisit comme composé de formule I la 3-méthallyl-5,5-diméthyl-thiazolidine-2,4-dione-2-(4-allyl-3-thiosemicarbazone) ou l'un de ses sels pharmaceutiquement utilisables.

12. Utilisation selon l'une des revendications 1 à 5, caractérisée en ce que l'on choisit comme composé de formule I la 3-méthallyl-thiazolidine-2,4-dione-2-(4-méthyl-3-thiosemicarbazone) ou l'un de ses sels pharmaceutiquement utilisables.

13. Utilisation selon l'une des revendications 1 à 5, caractérisée en ce que l'on choisit comme composé de formule I la 3-allyl-5-méthyl-thiazolidine-2,4-dione-2-(4-allyl-3-thiosemicarbazone) ou l'un de ses sels pharmaceutiquement utilisables.

14. Utilisation selon l'une des revendications 1 à 5, caractérisée en ce que l'on choisit comme composé de formule I la 3-méthallyl-5-méthyl-thiazolidine-2,4-dione-2-(4-méthyl-3-thiosemicarbazone) ou l'un de ses sels pharmaceutiquement utilisables.

15. Utilisation selon l'une des revendications 1 à 5, caractérisée en ce que l'on choisit comme composé de formule I la 3-propyl-thiazolidine-2,4-dione-2-(4-méthyl-3-thiosemicarbazone) ou l'un de ses sels pharmaceutiquement utilisables.

16. Utilisation selon l'une des revendications 1 à 5, caractérisée en ce que l'on choisit comme composé de formule I la 3-prop-2-yn-yl-thiazolidine-2,4-dione-2-(4-méthyl-3-thiosemicarbazone) ou l'un de ses sels pharmaceutiquement utilisables.

17. Utilisation selon l'une des revendications 1 à 5, caractérisée en ce que l'on choisit comme composé de formule I la 1,1'-dithiobis- {N-méthylformamide-3-méthallyl-4-oxo-5-méthyl-thiazolidinylidène}-hydrazone ou l'un de ses sels pharmaceutiquement utilisables.

18. Utilisation selon l'une des revendications 1 à 5, caractérisée en ce que l'on choisit comme composé de formule I la 1,1'-dithiobis-{N-allylformamide-3-allyl-4-oxo-5-méthyl-thiazolidinylidène}-hydrazone ou l'un de ses sels pharmaceutiquement utilisables.

19. Utilisation selon l'une des revendications 1 à 5, caractérisée en ce que l'on choisit comme composé de formule I la 1,1'-dithiohis- {N-méthylformamide-3-méthallyl-4-oxo-5,5-diméthyl-thiazolidinylidène}-hydrazone ou l'un de ses sels pharmaceutiquement utilisables.

20. Utilisation selon l'une des revendications 1 à 5, caractérisée en ce que l'on choisit comme composé de formule I la 1,1'-dithiobis-{N-allylformamide-3-méthallyl-4-oxo-5,5-diméthyl-thiazolidinylidène}-hydrazone ou l'un de ses sels pharmaceutiquement utilisables.

21. Utilisation selon l'une des revendications 1 à 5, caractérisée en ce que l'on choisit comme composé de formule I la 1,1'-dithiobis-{N-méthylformamide-3-allyl-4-oxo-thiazolidinylidène}-hydrazone ou l'un de ses sels pharmaceutiquement utilisables.

22. Utilisation selon l'une des revendications 1 à 5, caractérisée en ce que l'on choisit comme composé de formule I la 3-allyl-thiazolidine-2,4-dione-2-(4-allyl-3-thiosemicarbazone) ou l'un de ses sels pharmaceutiquement utilisables.

23. Utilisation selon l'une des revendications 1 à 5, caractérisée en ce que l'on choisit comme composé de formule I la 3-allyl-thiazolidine-2,4-dione-2-(4-propynyl-3-thiosemicarbazone) ou l'un de ses sels pharmaceutiquement utilisables.
